# EUROPÄISCHE PATENTSCHRIFT

(11) **EP 3 510 166 B1**
(45) Veröffentlichungstag und Bekanntmachung des Hinweises auf die Patenterteilung: **13.10.2021**
(21) Anmeldenummer: 17739924.3
(22) Anmeldetag: 05.07.2017
(51) Int. Cl.: G01N 33/48, C12N 5/071, C12Q 1/68, G01N 33/50

(54) **IN-VITRO VERFAHREN ZUR IDENTIFIZIERUNG UND ANALYSE VON PROTEINEN MIT STAMMZELLFUNKTION UNTER VERWENDUNG EINES DREIDIMENSIONALEN ZELLKULTURMODELLS DER SCHWEISSDRUESE**
IN VITRO METHOD FOR THE IDENTIFICATION AND ANALYSIS OF PROTEINS WITH STEM CELL FUNCTION USING A 3D CELL CULTURE MODEL OF THE SWEAT GLAND
PROCÉDÉ IN VITRO POUR L'IDENTIFICATION ET L'ANALYSE DE PROTÉINES À FONCTION DE CELLULES SOUCHES AU MOYEN D'UN MODÈLE DE CULTURE CELLULAIRE TRIDIMENSIONNEL DE LA GLANDE SUDORIPARE

(30) Priorität: 09.09.2016 DE 102016217172
(43) Veröffentlichungstag der Anmeldung: 17.07.2019
(73) Patentinhaber: Henkel AG & Co. KGaA, 40589 Düsseldorf (DE)
(72) Erfinder: KLAKA, Patricia, 51371 Leverkusen (DE); BANOWSKI, Bernhard, 40597 Düsseldorf (DE); GRÜDL, Sabine, 41812 Erkelenz (DE); WELSS, Thomas, 40591 Düsseldorf (DE); GIESEN, Melanie, 47608 Geldern (DE)
(86) Internationale Anmeldenummer: PCT/EP2017/066778
(87) Internationale Veröffentlichungsnummer: WO 2018/046162

(56) Entgegenhaltungen:
- LI HAIHONG ET AL: "Three-dimensional culture and identification of human eccrine sweat glands in matrigel basement membrane matrix", CELL AND TISSUE RESEARCH, SPRINGER, DE, Bd. 354, Nr. 3, 31. August 2013 (2013-08-31), Seiten 897-902, XP035331816, ISSN: 0302-766X, DOI: 10.1007/S00441-013-1718-3 [gefunden am 2013-08-31]
- KELSEA M. HUBKA ET AL: "Dissociative and Nondissociative Models for Culture of Human Eccrine Glands for Toxicology Testing and Tissue Engineering Applications", APPLIED IN VITRO TOXICOLOGY, Bd. 1, Nr. 3, 1. September 2015 (2015-09-01), Seiten 187-197, XP055399981, ISSN: 2332-1512, DOI: 10.1089/aivt.2015.0013
- HUANG ZHIJIAN ET AL: "Shh promotes sweat gland cell maturation in three-dimensional culture", CELL AND TISSUE BANKING, SPRINGER, NL, Bd. 17, Nr. 2, 23. Februar 2016 (2016-02-23), Seiten 317-325, XP035903320, ISSN: 1389-9333, DOI: 10.1007/S10561-016-9548-7 [gefunden am 2016-02-23]
- Viviana D. Robles-Munoz: "The Development of an in vitro 3D Histotypic Model of The Human Eccrine Sweat Gland", , 1. September 2014 (2014-09-01), XP055399433, Gefunden im Internet: URL:https://qmro.qmul.ac.uk/xmlui/bitstrea m/handle/123456789/12910/Robles, V. PhD Final 2016..pdf?sequence=4 [gefunden am 2017-08-17]
- ELEANOR KNIGHT ET AL: "Advances in 3D cell culture technologies enabling tissue-like structures to be created in vitro", JOURNAL OF ANATOMY., Bd. 227, Nr. 6, 20. November 2014 (2014-11-20), Seiten 746-756, XP055399546, GB ISSN: 0021-8782, DOI: 10.1111/joa.12257
- PATRICIA KLAKA ET AL: "A novel organotypic 3D sweat gland model with physiological functionality", PLOS ONE, Bd. 12, Nr. 8, 10. August 2017 (2017-08-10), Seite e0182752, XP055399221, DOI: 10.1371/journal.pone.0182752

## Beschreibung

Die vorliegende Erfindung betrifft ein in-vitro Verfahren zur Identifizierung und Analyse von Proteinen mit Stammzellfunktion, bei welchem zunächst ein dreidimensionales Schweißdrüsenäquivalent mit 500 bis 500.000 Schweißdrüsenzellen sowie einem Durchmesser von 100 bis 6.000 µm bereitgestellt und anschließend eine Identifizierung und Analyse von in diesem Äquivalent vorliegenden Proteinen mit Stammzellfunktion erfolgt. Die erfindungsgemäß verwendeten dreidimensionalen Schweißdrüsenäquivalente weisen geordnete Strukturen sowie unterschiedlich differenzierte Zellen auf und zeigen ein Reaktionsvermögen sowohl auf Genexpressionsebene als auch auf Ebene der Proteinexpression auf einen externen Stimulus, beispielsweise einen cholinergenen Stimulus durch Acetylcholin (auch als ACh bezeichnet).

Das Waschen, Reinigen und Pflegen des eigenen Körpers stellt ein menschliches Grundbedürfnis dar und die moderne Industrie versucht fortlaufend, diesen Bedürfnissen des Menschen in vielfältiger Weise gerecht zu werden. Besonders wichtig für die tägliche Hygiene ist die anhaltende Beseitigung oder zumindest Reduzierung des Körpergeruchs und der Achselnässe. Achselnässe und Körpergeruch entstehen durch die Sekretion aus ekkrinen und apokrinen Schweißdrüsen in der humanen Achselhöhle. Während die ekkrinen Drüsen der Thermoregulation des Körpers dienen und für die Entstehung der Achselnässe verantwortlich sind, scheiden die apokrinen Drüsen ein viskoses Sekret in Reaktion auf Stress ab, aus welchem durch bakterielle Zersetzung unangenehmer Körpergeruch entsteht.

Erste Forschungsarbeiten an nativen ekkrinen und apokrinen Schweißdrüsen wurden bereits Anfang des 20. Jahrhunderts durchgeführt, um diese zur Gruppe der exokrinen Drüsen gehörenden Hautanhangsgebilde zu klassifizieren. Schweißdrüsen lassen sich hiernach in apokrine und ekkrine Schweißdrüsen sowie eine Mischform aus apokrinen und ekkrinen Schweißdrüsen (auch als apoekkrine Schweißdrüse bezeichnet) einteilen. Die zuvor genannten Formen können anhand morphologischer und charakteristischer Merkmale unterschieden werden.

Die ekkrine Schweißdrüse, insbesondere die humane ekkrine Schweißdrüse, gehört zu den unverzweigten gewunden tubulären Drüsen und lässt sich in das sekretorische Endstück (auch als Coil bezeichnet), den dermalen Ausführgang (auch als Dukt bezeichnet) sowie den epidermalen Ausführgang (auch als Acrosyringium bezeichnet) unterteilen. Die in diesen Drüsenabschnitten vorhanden Zellen weisen unterschiedliche Aufgaben und Funktionen auf, wie beispielsweise die Sekretion im Coil, Rückresorption von Ionen im Dukt sowie Abgabe des Sekrets, insbesondere des Schweißes, an die umliegende Haut durch das Acrosyringium. Die ekkrinen Schweißdrüsen werden hauptsächlich durch den Neurotransmitter Acetylcholin (ACh) stimuliert, jedoch ist auch eine purinerge (beispielsweise mit ATP/UTP) sowie eine αβ-adrenerge (beispielsweise mit Noradrenalin) Stimulation möglich.

Im Hinblick auf die Vermeidung von Achselnässe und/oder Körpergeruch ist es daher wünschenswert, die Sekretion aus ekkrinen und/oder apokrinen Schweißdrüsen zu vermindern und/oder zu vermeiden. Dies kann beispielsweise dadurch erreicht werden, dass die Ausführungsgänge der ekkrinen Schweißdrüsen mittels sogenannter Plugs verstopft. Hierzu werden im Stand der Technik schweißhemmende Aluminium- und/oder Aluminium-Zirkoniumsalze eingesetzt, welche jedoch vom Verbraucher als kritisch empfunden werden. Weiterhin werden im Stand der Technik antibakterielle Mittel eingesetzt, welche den bakteriellen Abbau des Schweißes verhindern. Derartige Mittel können jedoch die natürliche Mikroflora der Haut unter der Achselhöhle negativ beeinflussen. Es ist daher wünschenswert, kosmetische Mittel bereitzustellen, welche in der Lage sind, die Achselnässe und/oder den Körpergeruch zuverlässig zu verhindern und welche frei von Aluminium- und/oder Aluminium-Zirkoniumsalzen sowie antibakteriell wirkenden Verbindungen sind. Eine Möglichkeit zur Bereitstellung derartiger Mittel besteht in dem Einsatz von Substanzen, welche die Stimulierung und/oder die biologischen Prozesse der Schweißdrüsen effektiv hemmen und somit die Schweißsekretion vermindern bzw. vermeiden. Um derartige Substanzen identifizieren zu können, können in-vivo Tests mit Versuchsteilnehmern durchgeführt werden. Solche Tests sind jedoch aufwändig und erlauben keine Screeningverfahren mit hohen Durchsatzraten. Zum anderen können in-vitro Tests unter Einsatz von Zellmodellen von Schweißdrüsen eingesetzt werden, an welchen der Einfluss von Testsubstanzen auf die Stimulation der Schweißdrüsen untersucht werden kann.

Damit die in-vitro erhaltenen Testergebnisse gut auf die in-vivo Situation übertragbar sind, muss das eingesetzte Zellmodell der Schweißdrüse die in-vivo-Situation möglichst genau nachbilden. Hierzu sind dreidimensionale Zellmodelle erforderlich, da die im Stand der Technik bekannten zweidimensionalen Modelle keine ausreichende physiologische Nähe zur nativen Schweißdrüse aufweisen und damit die in-vivo-Situation nur unzureichend wiedergeben. Darüber hinaus ist eine Aufklärung des Schweißsekretionsmechanismus erforderlich. Denn nur auf diese Weise können sogenannte biologische Targets, insbesondere durch die Schweißdrüsenzellen produzierte Proteine, identifiziert werden, deren Beeinflussung durch Testsubstanzen zu einer verminderten Schweißprodukten führt. Ein mögliches biologisches Target, welches im Zusammenhang mit der Schweißproduktion stehen könnte, sind multipotente Schweißdrüsenzellen, welche bereits im Zusammenhang mit Prozessen der Wundheilung bekannt sind.

Es besteht daher weiterhin ein Bedarf an in-vitro Verfahren, mit deren Hilfe biologische Targets, welche für eine erhöhte Schweißproduktion verantwortlich sind, identifiziert und analysiert werden können. Nach der Identifikation und Analyse derartiger Targets erfolgt bevorzugt die Untersuchung des Einflusses verschiedener Testsubstanzen auf diese Targets. Derartige in-vitro Verfahren sollen standardisierbar, kostengünstig und schnell durchführbar sein, so dass die Ermittlung des Einflusses von Testsubstanzen auf die biologischen Targets in Screeningverfahren mit hohen Durchsatzraten ermöglicht wird

In "Cell Tissue Research (2013) 354", wird auf den Seiten 897 bis 902 eine dreidimensionale Kultur von humanen eccrinen Schweißdrüsen in Matrigel Matrix beschrieben und ihre Charakterisierung mit Hilfe von Expressionsmarkern wie CK7, CK9, welche als Proteine mit Stammzellenfunktion angesehen werden, offenbart.

Die "Applied in vitro Toxicology (2015) 1 (3)" offenbart auf den Seiten 187 bis 197 drei Typen von dreidimensionalen Schweißdrüsenäquivalenten.

Der vorliegenden Erfindung lag daher die Aufgabe zugrunde, ein in-vitro Verfahren zur Identifikation und Analyse von Proteinen mit Stammzellenfunktion bereitzustellen, welches standardisierbar, kostengünstig und schnell durchführbar ist und dessen Ergebnisse sich auf die in-vivo-Situation übertragen lassen.

Es wurde nun überraschend gefunden, dass mit Hilfe bestimmter dreidimensionaler Schweißdrüsenäquivalente eine Identifikation und Analyse von Proteinen mit Stammzellenfunktion ermöglicht wird. Die eingesetzten dreidimensionalen Schweißdrüsenäquivalente weisen eine geordnete Struktur auf. Weiterhin bilden die primären Schweißdrüsenzellen dieser Äquivalente die gleichen Charakteristika aus wie native Schweißdrüsen. Daher lassen sich die mit diesen Äquivalenten erhaltenen Messdaten in Bezug auf die Identifikation und Analyse von Proteinen mit Stammzellenfunktion sehr gut auf die in-vivo-Situation übertragen.

Ein erster Gegenstand der vorliegenden Erfindung ist somit ein in-vitro Verfahren zur Identifikation und Analyse von Proteinen mit Stammzellfunktion in der humanen Schweißdrüse, umfassend die folgenden Verfahrensschritte:
a) Bereitstellen mindestens eines dreidimensionales Schweißdrüsenäquivalents, umfassend 500 bis 500.000 Schweißdrüsenzellen, wobei das mindestens eine dreidimensionale Schweißdrüsenäquivalent einen Durchmesser von 100 bis 6.000 µm aufweist und wobei das mindestens eine dreidimensionale Schweißdrüsenäquivalent frei von Matrixverbindungen und Trägern ist,
b) Identifizierung und Analyse von mindestens einem Protein mit Stammzellfunktion in dem in Verfahrensschritt a) bereitgestellten mindestens einen dreidimensionalen Schweißdrüsenäquivalent.

Die in dem erfindungsgemäßen Verfahren verwendeten dreidimensionalen Schweißdrüsenäquivalente bilden eine geordnete Struktur und weisen differenzierte Zellen mit den gleichen Charakteristika auf wie native Schweißdrüsen. Weiterhin zeigen diese Äquivalente eine Reaktion auf Genexpressions- als auch auf Proteinexpressionsebene auf einen Stimulus durch Acetylcholin (ACh). Die mit dem erfindungsgemäßen Verfahren erhaltenen Ergebnisse lassen sich daher gut auf die in-vivo-Situation übertragen. Durch Verwendung von kultivierten primären Schweißdrüsenzellen bei der Herstellung der Äquivalente kann eine hohe Standardisierung erreicht werden, da aus den kultivierten Zellen eine Vielzahl von Äquivalenten mit gleichen Eigenschaft erzeugt werden können. Weiterhin können durch den Einsatz von kultivierten primären Schweißdrüsenzellen Äquivalente mit annährend gleichen Zahlen an Schweißdrüsenzellen erzeugt werden, was ebenfalls eine hohe Standardisierbarkeit sicherstellt.

Unter dem Begriff "Protein mit Stammzellfunktion" werden erfindungsgemäß Proteine verstanden, welche von totipotenten und pluripotenten Stammzellen der dreidimensionalen Schweißdrüsenäquivalente gebildet werden und welche beispielsweise eine Rolle bei Wundheilungsprozessen spielen. Derartige Stammzellen sind nicht-spezialisierte, kontinuierlich proliferierende Zellen, welche über alle Informationen des Gesamtorganismus verfügen und sich daher unter geeigneten Bedingungen in mehr als einen Zelltyp entwickeln können. Sie dienen folglich der gewebespezifischen Zellregeneration, insbesondere bei der Wundheilung.

Weiterhin wird unter einem dreidimensionalen Schweißdrüsenäquivalent erfindungsgemäß ein Zellmodell aus Schweißdrüsenzellen verstanden, welches eine Ausdehnung in alle drei Raumrichtungen aufweist und in welchem die Zellen eine ähnliche Funktion, insbesondere eine identische Funktion, wie die Zellen einer nativen Schweißdrüse zeigen.

In Verfahrensschritt a) des erfindungsgemäßen Verfahrens wird zunächst mindestens ein dreidimensionales Schweißdrüsenäquivalent mit einer bestimmten Zellzahl und einem bestimmten Durchmesser bereitgestellt.

Besonders bevorzugte dreidimensionale Schweißdrüsenäquivalente weisen bestimmte Durchmesser auf. Es ist daher erfindungsgemäß vorteilhaft, wenn das in Verfahrensschritt a) bereitgestellte mindestens eine dreidimensionale Schweißdrüsenäquivalent einen Durchmesser von 100 bis 4.000 µm, vorzugsweise von 100 bis 2.000 µm, insbesondere von 200 bis 1.500 µm, aufweist. Der Durchmesser der bevorzugt kugelförmigen erfindungsgemäß verwendeten Schweißdrüsenäquivalente kann beispielsweise mittels mikroskopischer Vermessung unter Verwendung der Software "CellSens" bestimmt werden.

Im Rahmen der vorliegenden Erfindung ist es bevorzugt, wenn die in Verfahrensschritt a) verwendeten Schweißdrüsenäquivalente frei sind von Matrixverbindungen und Trägern. Unter Matrixverbindungen sind hierbei Verbindungen zu verstehen, welche zur Ausbildung räumlicher Netzwerke in der Lage sind. Hierunter fallen jedoch nicht die von den Zellen der Äquivalente selbst produzierten und ausgeschiedenen Substanzen, welche zur Ausbildung räumlicher Netzwerke in der Lage sind. Weiterhin werden unter Trägern im Sinne der vorliegenden Erfindung selbsttragende Stoffe verstanden, welche als Unterlage bzw. Gerüst für die Schweißdrüsenzellen dienen können. Gemäß einer bevorzugten Ausführungsform der vorliegenden Erfindung ist das in Verfahrensschritt a) bereitgestellte mindestens eine dreidimensionale Schweißdrüsenäquivalent frei von Matrixverbindungen und Trägern.

Unter dem Begriff "frei von" wird erfindungsgemäß verstanden, dass die dreidimensionalen Schweißdrüsenäquivalente weniger als 1 Gew.-%, bezogen auf das Gesamtgewicht des dreidimensionalen Schweißdrüsenäquivalents, an Matrixverbindungen und Träger enthalten. Es ist daher im Rahmen der vorliegenden Erfindung vorteilhaft, wenn die in Verfahrensschritt a) eingesetzten dreidimensionalen Schweißdrüsenäquivalente 0 bis 1 Gew.-%, vorzugsweise 0 bis 0,5 Gew.-%, bevorzugt 0 bis 0,2 Gew.-%, insbesondere 0 Gew.-%, jeweils bezogen auf das Gesamtgewicht des dreidimensionalen Schweißdrüsenäquivalents, an Matrixverbindungen und Trägern enthalten.

Besonders bevorzugt handelt es sich bei dem in Verfahrensschritt a) bereitgestellten dreidimensionalen Schweißdrüsenäquivalent um ein Äquivalent der ekkrinen und/oder apokrinen humanen Schweißdrüse. Bevorzugte Ausführungsformen der vorliegenden Erfindung sind daher dadurch gekennzeichnet, dass das in Verfahrensschritt a) bereitgestellte mindestens eine dreidimensionale Schweißdrüsenäquivalent ein dreidimensionales Schweißdrüsenäquivalent der ekkrinen und/oder apokrinen humanen Schweißdrüse ist. Derartige Schweißdrüsenäquivalente sind besonders gut zur Identifizierung und Analyse von Proteinen mit Stammzellfunktion sowie für die Ermittlung des Einflusses von Testsubstanzen auf diese Proteine geeignet.

Darüber hinaus ist es erfindungsgemäß besonders bevorzugt, wenn das in Verfahrensschritt a) bereitgestellte dreidimensionale Schweißdrüsenäquivalent aus humanen ekkrinen und/oder apokrinen Schweißdrüsen hergestellt wurde. Es ist daher im Rahmen der vorliegenden Erfindung vorteilhaft, wenn das in Verfahrensschritt a) bereitgestellte mindestens eine dreidimensionale Schweißdrüsenäquivalent ein aus ekkrinen und/oder apokrinen nativen humanen Schweißdrüsenzellen gewonnenes dreidimensionales Schweißdrüsenäquivalent ist.

Darüber hinaus hat es sich erfindungsgemäß als vorteilhaft erwiesen, wenn die in Verfahrensschritt a) bereitgestellten dreidimensionalen Schweißdrüsenäquivalente mindestens eine bestimme Zellart aufweisen. Der Einsatz derartiger Äquivalente führt zu einer besonders guten Identifizierung und Analyse von Proteinen mit Stammzellfunktion. Bevorzugt Ausführungsformen der vorliegenden Erfindung sind daher dadurch gekennzeichnet, dass das in Verfahrensschritt a) bereitgestellte mindestens eine dreidimensionale Schweißdrüsenäquivalent mindestens eine Zellart, ausgewählt aus der Gruppe von (i) Coilzellen, insbesondere clear cells, dark cells, sowie Myoepitheliumzellen, (ii) Duktzellen sowie (iii) deren Mischungen enthält. Unter dem Begriff "clear cells" werden erfindungsgemäß Zellen verstanden welche ein klares bzw. ungefärbtes Cytoplasma bei Anfärbung mit Farbstoffen, insbesondere mit Hematoxylin und Eosin aufweisen. Derartige "clear cells" sind bevorzugt sekretorische Zellen des Epitheliums, wobei die Plasmamembran an der apikalen und lateralen Oberfläche stark gefaltet ist. Das Cytoplasma dieser "clear cells" enthält hohe Mengen an Glycogen sowie viele Mitochondrien. Die Zellen befinden sich bevorzugt in Kontakt mit dem Lumen. Die wässrige Komponente des Schweißes, welche Elektrolyte und anorganische Substanzen enthält, wird bevorzugt von dieser Zellart ausgeschieden. Hingegen sind die zuvor angeführten "dark cells" Zellen, deren Vakuolen eine positive Anfärbung auf Mucopolysaccharidsäure aufweisen, deren Cytoplasma sich also durch Farbstoffe anfärben lässt. Diese "dark cells" stehen in Kontakt mit der Basalmembran und weisen im Vergleich zu den "clear cells" nur wenige Mitochondrien auf. Bevorzugt werden Makromoleküle, wie beispielsweise Glycoproteine, von diesen "dark cells" abgeschieden. Unter den zuvor angeführen "Myoepithelzellen" sind kontraktile Epithelzellen zu verstehen, welche ein Zytosklett mit sogennanten Gap junctions aufweisen und sich daher kontraktieren können. Auf diese Weise wird die Sekretabgabe aus den Drüsenendstücken unterstützt. Derartige Zellen befinden sich bevorzugt zwischen der Basalmembran und den zuvor angeführten "clear cells" und "dark cells". Schließlich werden unter dem Begriff "Duktzellen" erfindungsgemäß Zellen verstanden, welche die Wand des Duktes bilden und ein stratifiziertes kubisches Epithel aufweisen. Die zuvor angeführten Zellarten können durch neben der Verwendung von Hematoxylin und Eosin auch mittels immunzytochemischen Färbungen unter Einsatz von für diese Zellen spezifische Marker bestimmt werden. Ein für Myoepithelzellen einsetzbarer spezifischer Marker ist alpha-smooth muscle actin (auch als α-SMA bezeichnet). Als spezifische Marker "clear cells" eignet sich beispielsweise Substance P sowie S100. Weiterhin kann für "dark cells" der unter der Bezeichnung CGRP (calcitonin-gene related peptide) bekannte Marker, für Duktzellen die spezifischen Marker Cytokeratin 10 (auch als CK10 bezeichnet) und CD200 verwendet werden.

Im Folgenden sind besonders bevorzugte in Verfahrensschritt a) verwendete dreidimensionale Schweißdrüsenäquivalente beschrieben.

Eine besonders bevorzugte Ausführungsform dieses Erfindungsgegenstands ist demnach die Bereitstellung eines dreidimensionalen Schweißdrüsenäquivalents der ekkrinen und/oder apokrinen humanen Schweißdrüse, umfassend 500 bis 500.000 Schweißdrüsenzellen, wobei das dreidimensionale Schweißdrüsenäquivalent einen Durchmesser von 200 bis 1.500 µm aufweist.

Weiterhin ist eine besonders bevorzugte Ausführungsform dieses Erfindungsgegenstands die Bereitstellung eines aus ekkrinen und/oder apokrinen nativen humanen Schweißdrüsenzellen gewonnenes dreidimensionales Schweißdrüsenäquivalent, umfassend 500 bis 500.000 Schweißdrüsenzellen, wobei das dreidimensionale Schweißdrüsenäquivalent einen Durchmesser von 200 bis 1.500 µm aufweist.

Darüber hinaus ist eine besonders bevorzugte Ausführungsform dieses Erfindungsgegenstands die Bereitstellung eines dreidimensionalen Schweißdrüsenäquivalents, umfassend 500 bis 500.000 Schweißdrüsenzellen, wobei das dreidimensionale Schweißdrüsenäquivalent einen Durchmesser von 200 bis 1.500 µm aufweist und mindestens eine Zellart, ausgewählt aus der Gruppe von clear cells, dark cells, Myoepitheliumzellen, Duktzellen sowie deren Mischungen enthält.

Zudem ist eine besonders bevorzugte Ausführungsform dieses Erfindungsgegenstands die Bereitstellung eines aus ekkrinen und/oder apokrinen nativen humanen Schweißdrüsenzellen gewonnenes dreidimensionales Schweißdrüsenäquivalent, umfassend 500 bis 500.000 Schweißdrüsenzellen, wobei das dreidimensionale Schweißdrüsenäquivalent einen Durchmesser von 200 bis 1.500 µm aufweist und mindestens eine Zellart, ausgewählt aus der Gruppe von clear cells, dark cells, Myoepitheliumzellen, Duktzellen sowie deren Mischungen enthält.

Weiterhin ist eine besonders bevorzugte Ausführungsform dieses Erfindungsgegenstands die Bereitstellung eines dreidimensionalen Schweißdrüsenäquivalents der ekkrinen und/oder apokrinen humanen Schweißdrüse, umfassend 500 bis 500.000 Schweißdrüsenzellen, wobei das dreidimensionale Schweißdrüsenäquivalent einen Durchmesser von 200 bis 1.500 µm aufweist und 0 Gew.-%, bezogen auf das Gesamtgewicht des dreidimensionalen Schweißdrüsenäquivalents, an Matrixverbindungen und Trägern enthält.

Zudem ist eine besonders bevorzugte Ausführungsform dieses Erfindungsgegenstands die Bereitstellung eines dreidimensionalen Schweißdrüsenäquivalents der ekkrinen und/oder apokrinen humanen Schweißdrüse, umfassend 500 bis 500.000 Schweißdrüsenzellen, wobei das dreidimensionale Schweißdrüsenäquivalent einen Durchmesser von 200 bis 1.500 µm aufweist und 0 Gew.-%, bezogen auf das Gesamtgewicht des dreidimensionalen Schweißdrüsenäquivalents, an Matrixverbindungen und Trägern enthält, wobei die Matrixverbindungen und/oder Träger sind aus der Gruppe von Collagenen, insbesondere Collagen Typ I und/oder Typ III und/oder Typ IV, Scleroproteinen, Gelatinen, Chitosanen, Glucosaminen, Glucosaminoglucanen (GAG), Heparinsulfatproteoglucanen, sulfatierten Glycoproteinen, Wachstumsfaktoren, vernetzten Polysacchariden, vernetzten Polypeptiden sowie deren Mischungen.

Die in dem Verfahrensschritt a) bereitgestellten dreidimensionalen Schweißdrüsenäquivalente weisen eine höhere Standardisierbarkeit und Verfügbarkeit auf wie isolierte Schweißdrüsen und besitzen eine größere Nähe zur in-vivo Situation als eindimensionale und zweidimensionale Schweißdrüsenmodelle. Weiterhin stellen diese Äquivalente eine kostengünstige Alternative zu in-vivo Studien am Menschen dar, da mittels dieser Äquivalente Proteine mit Stammzellfunktion identifiziert sowie deren Einfluss auf die Schweißsekretion analysiert werden können. Denn die dreidimensionalen Schweißdrüsenäquivalente simulieren die Schweißdrüse in-vivo sowohl in ihrer Struktur als auch in ihrer histologischen Zusammensetzung, so dass die mit diesen Äquivalenten erhaltenen Informationen gut auf den Menschen übertragbar sind.

Die in Verfahrensschritt a) bereitgestellten dreidimensionalen Schweißdrüsenäquivalente lassen sich beispielsweise durch das folgende Herstellungsverfahren erhalten.

In einem ersten Schritt werden zunächst isolierte Schweißdrüsen bereitgestellt, welche aus Hautbiopsien oder dergleichen erhalten werden können und welche aus ihrer natürlichen Umgebung entfernt wurden. Bevorzugt werden die isolierten Schweißdrüsen des ersten Schritts durch Isolierung nativer Schweißdrüsen, insbesondere nativer ekkriner und/oder apokriner Schweißdrüsen, aus der menschlichen Haut erhalten, wobei die Isolierung der nativen Schweißdrüsen bevorzugt durch enzymatischen Verdau der menschlichen Haut unter Verwendung einer Mischung aus 2 bis 3 mg/ml Collagenase II und 0,1 bis 0,2 mg/ml Thermolysin für 3 bis 6 Stunden bei 35 bis 40 °C, insbesondere bei 37°C, erfolgt.

Diese isolierten Schweißdrüsen werden in einem zweiten Schritt in einem bestimmten Nährmedium kultiviert, um eine Zellkultur zu erhalten. Eine besonders gute Kultivierung der in dem ersten Schritt erhaltenen isolierten Schweißdrüsenzellen wird erreicht, wenn eine Mischung aus DMEM und Ham's F12 im Gewichtsverhältnis 3:1, welche zusätzlich 10 Gew.-%, bezogen auf das Gesamtgewicht der Mischung, fetales Kälber Serum (FCS), enthält, als Nährmedium verwendet wird. Die Kultivierung dieser Zellen in dem zuvor beschriebenen Nährmedium erfolgt vorzugsweise für 7 bis 28 Tage, insbesondere für 14 Tage, bei einer Temperatur von 36 bis 38 °C und einem CO₂-Gehalt von 5 Gew.-%, bezogen auf das Gesamtgewicht der zur Kultivierung eingesetzten Atmosphäre.

Aus den kultivierten Zellen wird im dritten Schritt eine Zellpräparation von primären Schweißdrüsenzellen in einem Nährmedium hergestellt, wobei die Zellzahl der primären Schweißdrüsenzellen in der Zellpräparation 50 bis 250.00 Zellen pro µL, vorzugsweise 100 bis 10.000 Zellen pro µL, bevorzugt 150 bis 5.000 Zellen pro µL, weiter bevorzugt 200 bis 3.200 Zellen pro µL, noch weiter bevorzugt 300 bis 1.000 Zellen pro µL, insbesondere 400 bis 600 Zellen pro µL Nährmedium beträgt. Die Zellpräparation von primären Schweißdrüsenzellen wird bevorzugt durch Ablösung der im zweiten Schritt kultivierten Schweißdrüsenzellen, insbesondere durch schonende Trypsinierung, Kultivierung dieser abgelösten Schweißdrüsenzellen in Monolayer-Kulturen, Suspendierung der kultivierten primären Schweißdrüsenzellen in einem Nährmedium sowie Einstellung der Zellzahl hergestellt. Für die Kultivierung der abgelösten Schweißdrüsenzellen sowie zur Herstellung der Zellsuspension hat es sich als vorteilhaft erwiesen, wenn eine Mischung aus DMEM und Ham's F12 im Gewichtsverhältnis 3:1, welche zusätzlich 10 Gew.-%, bezogen auf das Gesamtgewicht der Mischung, fetales Kälber Serum (FCS), enthält, als Nährmedium verwendet wird. Die Kultivierung der abgelösten Schweißdrüsenzellen wird vorzugsweise bei einer Temperatur von 36 bis 38 °C und einem CO₂-Gehalt von 5 Gew.-%, bezogen auf das Gesamtgewicht der zur Kultivierung eingesetzten Atmosphäre, bis zur Kofluenz durchgeführt.

Anschließend werden in einem vierten Schritt 10 bis 100 µL, vorzugsweise von 20 bis 80 µL, bevorzugt von 30 bis 70 µL, insbesondere von 40 bis 60 µL dieser Zellpräparation in hängenden Zustand, also in Form eines von einer Oberfläche frei schwebend herabhängenden Tropfens, kultiviert, bis sich die dreidimensionalen Schweißdrüsenäquivalente ausgebildet haben. In diesem Zusammenhang hat sich die Verwendung sogenannter Hanging-Drop-Wells, wie beispielsweise in der Offenlegungsschrift WO 2012/014047 A1 offenbart und kommerziell von der Firma Insphero als GravityPLUS®-Einsaatplatte mit SureDrop® Inlet-Einführsystem sowie GravityTRAP®-Ernteplatte erhältlich, als vorteilhaft erwiesen. Bevorzugt erfolgt die Kultivierung der Zellpräparation in hängendem Zustand für einen Zeitraum von 1 bis 25 Tagen, insbesondere von 2 bis 7 Tagen, bei einer Temperatur von 36 bis 38 °C und einem CO₂-Gehalt von 5 Gew.-%, bezogen auf das Gesamtgewicht der zur Kultivierung eingesetzten Atmosphäre. Hierbei ist es bevorzugt, wenn während der Kultivierungsdauer, insbesondere nach 1 bis 3 Tagen, 40 Volumenprozent, bezogen auf das Gesamtvolumen der zuvor angeführten Zellpräparation, des Nährmediums der Zellpräparation durch frisches Nährmedium ersetzt werden.

Nach Isolation der erhaltenen Äquivalente durch Zugabe von 50 bis 200 µL, insbesondere von 70 bis 100 µL, Nährmedium können die Äquivalente direkt für den Verfahrensschritt b) des erfindungsgemäßen Verfahrens verwendet oder erneut kultiviert werden. Die erneute Kultivierung der erhaltenen Äquivalente erfolgt bevorzugt für einen Zeitraum von 1 bis 6 Tagen bei einer Temperatur von 36 bis 38 °C und einem CO₂-Gehalt von 5 Gew.-%, bezogen auf das Gesamtgewicht der zur Kultivierung eingesetzten Atmosphäre.

Die Bereitstellung der dreidimensionalen Schweißdrüsenäquivalente in Verfahrensschritt a) erfolgt daher besonders bevorzugt mit den nachfolgend beschriebenen Verfahren, welches die folgenden Schritte in der angegebenen Reihenfolge umfasst:
(i) Bereitstellung von isolierten Schweißdrüsen, wobei die isolierten Schweißdrüsen durch Isolierung nativer ekkriner und/oder apokriner Schweißdrüsen aus der menschlichen Haut und anschließender Suspendierung dieser isolierten Schweißdrüsen in Nährmedium erhalten werden,
(ii) Bereitstellung einer Zellpräparation von primären Schweißdrüsenzellen aus den in Verfahrensschritt (i) isolierten Schweißdrüsen, wobei die Zellzahl der primären Schweißdrüsenzellen in der Zellpräparation 400 bis 600 Zellen pro µL beträgt und wobei die Zellpräparation von primären Schweißdrüsenzellen ein Volumen von 40 bis 60 µL aufweist,
(iii) Kultivierung der in Verfahrensschritt (ii) bereitgestellten Zellpräparation in einem hängenden Zustand, wobei der hängende Zustand der Zellpräparation durch Verwendung einer Hanging-Drop-Multiwellplatte erreicht wird und wobei während der Kultivierungsdauer 40 Volumenprozent, bezogen auf das Gesamtvolumen der in diesem Verfahrensschritt verwendeten Zellpräparation, des Nährmediums der Zellpräparation durch frisches Nährmedium ersetzt werden,
(iv) Isolierung des in Verfahrensschritts (iii) erhaltenen dreidimensionalen Schweißdrüsen-äquivalents, wobei die Isolierung des dreidimensionalen Schweißdrüsenäquivalents durch Zugabe von 50 bis 200 µL Nährmedium zur Ablösung des Modells erfolgt,
(v) Gegebenenfalls Kultivierung des in Verfahrensschritt (iv) isolierten dreidimensionalen Schweißdrüsenäquivalents für einen Zeitraum von 1 bis 6 Tagen bei einer Temperatur von 36 bis 38 °C und einem CO₂-Gehalt von 5 Gew.-%, bezogen auf das Gesamtgewicht der zur Kultivierung eingesetzten Atmosphäre.

Da im Rahmen der vorliegenden Erfindung bevorzugt die Identifikation und Analyse von Proteinen mit Stammzellfunktion der ekkrinen und/oder apokrinen humanen Schweißdrüse erfolgen soll, erfolgt die Herstellung der in Schritt a) bereitgestellten Äquivalente unter Verwendung von ekkrinen und/oder apokrinen nativen humanen Schweißdrüsen. Unter ekkrinen und/oder apokrinen nativen Schweißdrüsen sind hierbei ekkrine und/oder apokrine Schweißdrüsen zu verstehen, welche aus der Haut des Menschen, insbesondere aus Hautbiopsien des Menschen oder durch andere Verfahren, isoliert wurden. Weiterhin werden die in Schritt a) bereitgestellten dreidimensionalen Schweißdrüsenäquivalente bevorzugt ausschließlich unter Verwendung von in-vitro Methoden hergestellt. Folglich sind keine Verfahrensschritte enthalten, bei welchen in-vivo Methoden eingesetzt werden. Somit können diese Äquivalente auch zur Testung von Substanzen verwendet werden, welche zur kosmetischen Anwendung vorgesehen sind. Weiterhin erlaubt dieses Herstellungsverfahren eine kostengünstige Herstellung von standardisierten Äquivalenten, welche in Screeningverfahren mit hohen Durchsatzraten eingesetzt werden können. Zudem resultiert dieses Herstellverfahren in dreidimensionalen Schweißdrüsenäquivalenten, welche geordnete Strukturen ausbilden, unterschiedlich differenzierte Zellen aufweisen und Schweißdrüsenspezifische Marker exprimieren, so dass eine gute Übertragbarkeit von in-vitro Daten auf die in-vivo-Situation ermöglicht wird.

Ein Herstellverfahren für die in Verfahrensschritt a) des erfindungsgemäßen Verfahrens bereitgestellten dreidimensionalen Schweißdrüsenäquivalente ist beispielsweise in der deutschen Anmeldung DE 10 2015 222 279 offenbart.

In dem zweiten Verfahrensschritt des erfindungsgemäßen Verfahrens erfolgt die Identifizierung und Analyse mindestens eines Proteins mit Stammzellenfunktion in dem in Verfahrensschritt a) bereitgestellten dreidimensionalen Schweißdrüsenäquivalent.

Im Rahmen der vorliegenden Erfindung werden vorteilhafterweise bestimmte Proteine mit Stammzellfunktion identifiziert und analysiert. Bevorzugte Ausführungsformen der vorliegenden Erfindung sind daher dadurch gekennzeichnet, dass das mindestens eine Protein mit Stammzellfunktion in Verfahrensschritt b) ausgewählt ist aus der Gruppe von Strukturproteinen, Signallingproteinen, Zellproliferationsproteinen, Zelladhäsionsproteinen sowie deren Mischungen. Unter Strukturproteinen werden erfindungsgemäß Proteine bezeichnet, welche als Gerüststoff in Zellen dienen und maßgeblich an der Ausbildung von Fasern durch Aggregation von Monomeren Proteinsträngen beteiligt sind. Sie werden daher auch als Skleroproteine, Faserproteine oder Gerüstproteine bezeichnet und dienen zur Stabilisierung der Form, Festigkeit und Elastizität von Zellen. Unter dem Begriff "Signallingproteine" werden erfindungsgemäß Proteine verstanden, welche durch Interaktion mit dem Rezeptor einer Zielzelle oder durch Eindringen in die Zelle durch die Zellmembran Signale zu der Zielzelle übertragen. Nach Aktivierung der Zielzelle erfolgt zumeist die Aktivierung von sogenannten "second messengers", welche zu verschiedenen physiologischen Effekten führen. Signallingproteine können beispielsweise ausgewählt sein aus Proteinen mit Lipidresten, Phospholipiden, Aminosäuren, Monoaminen, Proteinen, Glycoproteinen oder Gasen. Während Signallingproteine, welche an Rezeptoren auf der Zelloberfläche binden, zumeist ein hohes Molekulargewicht aufweisen und hydrophil sind, weisen die in die Zelle eindringenden Signallingproteine meist geringe Molekulargewichte auf und sind hydrophob. Zellproliferationsproteine sind erfindungsgemäß Proteine, welche die Zellproliferation steuern, insbesondere erhöhen oder vermindern. Unter Zellproliferation wird hierbei das Ansteigen der Zellzahl aufgrund von Zellwachstum und Zellteilung verstanden. Hingegen sind Zelladhäsionsproteine im Rahmen der vorliegenden Erfindung Proteine, welche an der Zelloberfläche lokalisiert sind und für die Bindung zu anderen Zellen oder zu der extrazellulären Matrix verantwortlich sind. Durch diese Proteine erfolgen daher eine Bindung der Zellen untereinander sowie eine Bindung der Zellen zu ihrer Umgebung. Derartige Proteine weisen bevorzugt drei Domänen auf, intrazelluläre Domäne, die transmembrane Domäne sowie die extrazelluläre Domäne. Während die intrazelluläre Domäne bevorzugt mit dem Zytoskelet wechselwirkt bindet die extrazelluläre Domäne entweder an andere Zelladhäsionsproteine der gleichen Art oder mit Zellädhäsionsproteinen der extrazellulären Matrix.

Die zuvor genannten Proteine besitzen nicht nur einen Einfluss auf die Wundheilung, sondern können auch die Schweißsekretion beeinflussen. Daher eignen sich diese Proteine besonders als biologische Targets zur Untersuchung des Sekretionsmechanismus.

Die Identifizierung und Analyse des Proteins mit Stammzellfunktion, insbesondere der zuvor angeführten Proteine, wird erfindungsgemäß unter Verwendung bestimmter Methoden durchgeführt. Es ist daher erfindungsgemäß bevorzugt, wenn die Identifizierung und Analyse in Verfahrensschritt b) mit Methoden, ausgewählt aus der Gruppe von molekularbiologischen Methoden, Proteinanalysen, Assays zur Bestimmung der Funktionalität sowie deren Kombinationen erfolgt. Im Rahmen der vorliegenden Erfindung einsetzbare molekularbiologische Methoden sind beispielsweise die NGS (next generation sequencing)-Analyse sowie die qRT-PCR (quantitative real-time PCR). Mittels dieser Methoden können die zuvor angeführten Proteine mittels Genexpressionsanalysen identifiziert und quantitativ bestimmt werden. Die in den dreidimensionalen Schweißdrüsenäquivalenten erhaltenen Expressionslevel der Proteine wurden mit dem Expressionslevel dieser Proteine in Proben der humanen Schweißdrüse sowie in Proben der Vollhaut verglichen. Das Expressionslevel dieser Proteine in den dreidimensionalen Schweißdrüsenäquivalenten sowie in der humanen Schweißdrüse war signifikant höher als in den Proben der Vollhaut, so dass diese Proteine daher spezifische Markerproteine für die Schweißdrüse darstellen können. Weiterhin war die erhaltene Expression dieser Proteine in den dreidimensionalen Schweißdrüsenäquivalenten vergleichbar mit der Expression dieser Proteine in der humanen Schweißdrüsen. Die in dem erfindungsgemäßen Verfahren eingesetzten Schweißdrüsenäquivalente bilden daher die Situation in-vivo hervorragend ab und gewährleisten auf diese Weise eine gute Übertragbarkeit der in-vitro-Ergebnisse auf die in-vivo-Situation.

Geeignete Proteinanalysen sind beispielsweise Immunmarkierungen der zuvor angeführten Proteine mittels spezifischer Marker, wie die Methode der Immunfluoreszenz, Western Blot-Analysen und/oder ELISA. Mit den beiden zuletzt genannten Methoden ist ebenfalls eine quantitative Bestimmung der zuvor genannten Proteine möglich.

Im Rahmen des erfindungsgemäßen Verfahrens hat es sich als vorteilhaft erwiesen, wenn nach dem Verfahrensschritt b) ein weiterer Verfahrensschritt c) durchgeführt wird. In diesem Verfahrensschritt c) wird der Einfluss von verschiedenen Testsubstanzen auf die in Verfahrensschritt b) identifizierten Proteine mit Stammzellfunktion, insbesondere die zuvor angeführten bestimmten Proteine, ermittelt. Bevorzugte Ausführungsformen der vorliegenden Erfindung sind daher dadurch gekennzeichnet, dass in einem zusätzlichen Verfahrensschritt c) der Einfluss von Verbindungen auf die in Verfahrensschritt b) identifizierten Proteine mit Stammzellaktivität untersucht wird. Bei denen in Verfahrensschritt c) eingesetzte Verbindungen handelt es sich bevorzugt um Inhibitoren dieser Proteine, falls die Proteine die Schweißsekretion erhöhen. Vermindern die zuvor genannten Proteine jedoch die Schweißsekretion, werden als Verbindungen in Verfahrensschritt c) bevorzugt Aktivatoren eingesetzt. In diesem Verfahrensschritt kann neben dem Einfluss der Verbindungen auf die Schweißsekretion auch der Einfluss dieser Verbindungen auf die Stammzellaktivität und die Wundheilung untersucht werden.

In diesem Zusammenhang ist es bevorzugt, wenn in Verfahrensschritt c) bestimmte Methoden zur Ermittlung des Einflusses der Verbindung auf die in Verfahrensschritt b) identifizierten Proteine eingesetzt werden. Es ist daher erfindungsgemäße vorteilhaft, wenn der Einfluss der mindestens einen Verbindung in Verfahrensschritt c) mit Methoden, ausgewählt aus der Gruppe von molekularbiologischen Methoden, Proteinanalysen, Assays zur Bestimmung der Funktionalität sowie deren Kombinationen erfolgt. Bezüglich der Methoden wird auf die vorstehend genannten und in Verfahrensschritt b) verwendeten Methoden verwiesen, welche für die Durchführung des Verfahrensschritts c) gleichermaßen herangezogen werden können.

Die folgenden Beispiele erläutern die vorliegende Erfindung, ohne sie jedoch darauf einzuschränken:

### Beispiele:

### 1 Bereitstellung der dreidimensionalen Schweißdrüsenäquivalente (Verfahrensschritt a))

### 1.1 Isolation der Schweißdrüsen

Die nativen Schweißdrüsen wurden aus humanen Gewebeproben, sogenannten Biopsien gewonnen, welche aus plastischen Operationen von Patienten stammen, die der Verwendung des Materials zu Forschungszwecken zugestimmt haben. Das verwendete Gewebe wurde bei Oberarmstraffungen und Gesichtsstraffungen entnommen. Hieraus wurden die ekkrinen und apokrinen Schweißdrüsen aus dem Achselbereich isoliert.

Hierzu wurde die jeweilige Biopsie in kleine Stücke zerteilt und danach in Stücke von maximal ca. 1 cm x 1 cm geschnitten wurde. Anschließend erfolgte der Verdau der Haut mit einem Gemisch aus gleichen Teilen Collagenase II (5 mg/ml) und Thermolysin (0,25 mg/ml) bei 37 °C im Brutschrank für ca. 3,5 bis 5 Stunden, bis das Bindegewebe fast vollständig verdaut war. Diese Mischung wurde anschließend bei 1200 rpm für 5 Minuten zentrifugiert und der Überstand verworfen, um die Enzymlösung sowie das überschüssige Fett zu entfernen. Das entstandene Pellet wurde in DMEM-Lösung aufgenommen und die Lösung in eine Petrischale überführt. Anhand einer Mikrokapillare wurden intakte Schweißdrüsen unter einem Binokular isoliert und in frisches DMEM-Medium überführt.

### 1.2 Kultivieren der isolierten nativen Schweißdrüsen

Die in Schritt 1.1 isolierten Schweißdrüsen wurden in Collagen-I beschichtete Kulturflaschen gegeben und anschließend 25 ml Nährmedium hinzugefügt. Nach Kultivierung für 7 bis 21 Tage im Brutschrank bei 37 °C und 5 % CO₂ wurden die auswachsenden Schweißdrüsenzellen abgelöst und auf Collagen-I beschichteten Kulturflaschen erneut bis zur Konfluenz kultiviert (Monolayer-Kultur der primären Schweißdrüsenzellen).

**Die Zusammensetzung des eingesetzten Nährmediums ist wie folgt:**

| | |
|---|---|
| Bestandteile des Mediums | |
| DMEM/ Ham's F12 Nutrient Mix | 3 : 1 |
| Fätales Kälber Serum (FCS) | 10% |
| EGF | 10 ng/ml |
| Hydrocortison | 0,4 µg/ml |
| Insulin | 0,12 Ul/ml |
| Choleratoxin | 10⁻¹⁰ M |
| Adenin | 2,43 g/ml |
| Gentamicin | 25 µg/ml |
| Penicillin G | 100 Ul/ml |
| Thiodothyronin | 2*10⁻⁹ M |
| Ascorbyl-2-phosphat | 1 mM |

### 1.3 Herstellung der Zellpräparation und der dreidimensionalen Schweißdrüsenäquivalente

Nach Bestimmung der genauen Zellzahlen der obigen Monolayer-Kulturen der primären Schweißdrüsenzellen wurden diese unter Verwendung des obigen Nährmediums auf eine Zellzahl von 10 bis 5.000 Zellen pro µl eingestellt und anschließend je 50 µl dieser Zellsuspension mittels des "SureDrop® Inlet"-Einführsystems in Wells einer "GravityPLUS®"-Einsaatplatte (jeweils Firma Inshpero AG, Schweiz) überführt. Die Kultivierung erfolgt bei 36 bis 38 °C und einem CO₂-Gehalt von 5 Gew.-%, bezogen auf das Gesamtgewicht der zur Kultivierung eingesetzten Atmosphäre. Nach 1 bis 3 Tagen werden jeweils 40 Vol.-% des Mediums in den Wells der "GravityPLUS®"-Einsaatplatte durch frisches Nährmedium ersetzt. Nach 2 bis 7 Tagen Kultivierung werden die 3D-Schweißdrüsenäquivalente durch Zugabe von 50 bis 200 µL Nährmedium geerntet und in eine "GravityTRAP®"-Platte (Firma Insphero AG, Schweiz) überführt. Vor der Ernte wird die "GravityTRAP®"-Platte mit 60 bis 100 µL Keratinozyten-Medium mithilfe einer Multikanalpipette angefeuchtet, um die Bildung von Luftblasen zu minimieren und den Verlust der dreidimensionalen Schweißdrüsenäquivalente zu vermeiden. Nach der Ernte wird die Platte für 1 bis 5 Minuten bei 100 bis 300 xg zentrifugiert, um Luftblasen zu entfernen. Ein Teil der dreidimensionalen Schweißdrüsenäquivalente wurde analysiert, wohingegen ein weiterer Teil für weitere 1 bis 6 Tage in den Vertiefungen der Ernteplatte bei 37 °C und 5 Gew.-% CO₂, bezogen auf das Gesamtgewicht der zur Kultivierung eingesetzten Atmosphäre, kultiviert wurde.

### 2. Identifizierung und Analyse eines Proteins mit Stammzellfunktion (Verfahrensschritt b))

Der Nachweis der zuvor genannten Strukturproteinen, Signallingproteinen, Zellproliferationsproteinen, Zelladhäsionsproteinen kann beispielsweise mittels molekularbiologischer Methoden bestimmt werden. Hierbei wird zunächst die mRNA mithilfe des "RNeasy Micro Kits" (Qiagen) nach Herstellerangaben isoliert und anschließend mittels quantitativer Real-Time-PCR analysiert (Bellas et. al.: "In Vitro 3D Full-Thickness Skin-Equivalent Tissue Model Using Silk and Collagen Biomaterials"; Macromolecular Bioscience, 2012, 12, Seiten 1627-1636). Es ist jedoch auch möglich, die zuvor genannten Proteine mit Stammzellfunktion mithilfe von Immunofluoreszenzfärbungen nachzuweisen. So konnte beispielsweise das Strukturprotein (auch als Intermediärfilament bezeichnet) Nestin in den in Verfahrensschritt a) bereitgestellten dreidimensionalen Schweißdrüsenäquivalenten mittels dieses Verfahrens nachgewiesen werden. Nestin wird in 90% aller von Schweißdrüsen abgeleiteten Stammzellen (auch als SCSCs bezeichnet) exprimiert und ist daher ein Hinweis auf das Vorliegen von Stammzellen in den dreidimensionalen Schweißdrüsenäquivalenten.

## Patentansprüche

1. In-vitro Verfahren zur Identifikation und Analyse von Proteinen mit Stammzellfunktion in der humanen Schweißdrüse, umfassend die folgenden Verfahrensschritte:
a) Bereitstellen mindestens eines dreidimensionales Schweißdrüsenäquivalents, umfassend 500 bis 500.000 Schweißdrüsenzellen, wobei das mindestens eine dreidimensionale Schweißdrüsenäquivalent einen Durchmesser von 100 bis 6.000 µm aufweist und wobei das mindestens eine dreidimensionale Schweißdrüsenäquivalent frei von Matrixverbindungen und Trägern ist,
b) Identifizierung und Analyse von mindestens einem Protein mit Stammzellfunktion in dem in Verfahrensschritt a) bereitgestellten mindestens einen dreidimensionalen Schweißdrüsenäquivalent.

2. Verfahren nach Anspruch 1, **dadurch gekennzeichnet, dass** das in Verfahrensschritt a) bereitgestellte mindestens eine dreidimensionale Schweißdrüsenäquivalent einen Durchmesser von 100 bis 4.000 µm, vorzugsweise von 100 bis 2.000 µm, insbesondere von 200 bis 1.500 µm, aufweist.

3. Verfahren nach einem der vorangehenden Ansprüche, **dadurch gekennzeichnet, dass** das in Verfahrensschritt a) bereitgestellte mindestens eine dreidimensionale Schweißdrüsenäquivalent ein dreidimensionales Schweißdrüsenäquivalent der ekkrinen und/oder apokrinen humanen Schweißdrüse ist.

4. Verfahren nach einem der vorangehenden Ansprüche, **dadurch gekennzeichnet, dass** das in Verfahrensschritt a) bereitgestellte mindestens eine dreidimensionale Schweißdrüsenäquivalent mindestens eine Zellart, ausgewählt aus der Gruppe von (i) Coilzellen, insbesondere clear cells, dark cells sowie Myoepitheliumzellen, (ii) Duktzellen sowie (iii) deren Mischungen enthält.

5. Verfahren nach einem der vorangehenden Ansprüche, **dadurch gekennzeichnet, dass** das mindestens eine Protein mit Stammzellfunktion in Verfahrensschritt b) ausgewählt ist aus der Gruppe von Strukturproteinen, Signallingproteinen, Zellproliferationsproteinen, Zelladhäsionsproteinen sowie deren Mischungen.

6. Verfahren nach einem der vorangehenden Ansprüche, **dadurch gekennzeichnet, dass** die Identifizierung und Analyse in Verfahrensschritt b) mit Methoden, ausgewählt aus der Gruppe von molekularbiologischen Methoden, Proteinanalysen, Assays zur Bestimmung der Funktionalität sowie deren Kombinationen erfolgt.

7. Verfahren nach einem der vorangehenden Ansprüche, **dadurch gekennzeichnet, dass** in einem zusätzlichen Verfahrensschritt c) der Einfluss von Verbindungen auf die in Verfahrensschritt b) identifizierten Proteine mit Stammzellaktivität untersucht wird.

8. Verfahren nach Anspruch 7, **dadurch gekennzeichnet, dass** der Einfluss der mindestens einen Verbindung in Verfahrensschritt c) mit Methoden, ausgewählt aus der Gruppe von molekularbiologischen Methoden, Proteinanalysen, Assays zur Bestimmung der Funktionalität sowie deren Kombinationen erfolgt.

## Claims

1. An in vitro method for identifying and analyzing proteins having a stem cell function in the human sweat gland, comprising the following method steps:
a) providing at least one three-dimensional sweat gland equivalent, comprising 500 to 500,000 sweat gland cells, wherein the at least one three-dimensional sweat gland equivalent has a diameter of from 100 to 6,000 µm, and wherein the at least one three-dimensional sweat gland equivalent is free of matrix compounds and carriers,
b) identifying and analyzing at least one protein having a stem cell function in the at least one three-dimensional sweat gland equivalent provided in method step a).

2. The method according to claim 1, **characterized in that** the at least one three-dimensional sweat gland equivalent provided in method step a) has a diameter of from 100 to 4,000 µm, preferably from 100 to 2,000 µm, in particular from 200 to 1,500 µm.

3. The method according to one of the preceding claims, **characterized in that** the at least one three-dimensional sweat gland equivalent provided in method step a) is a three-dimensional sweat gland equivalent of the eccrine and/or apocrine human sweat gland.

4. The method according to one of the preceding claims, **characterized in that** the at least one three-dimensional sweat gland equivalent provided in method step a) contains at least one cell type selected from the group of (i) coil cells, in particular clear cells, dark cells and myoepithelial cells, (ii) duct cells and (iii) mixtures thereof.

5. The method according to one of the preceding claims, **characterized in that** the at least one protein having a stem cell function in method step b) is selected from the group of structural proteins, signaling proteins, cell proliferation proteins, cell adhesion proteins and mixtures thereof.

6. The method according to one of the preceding claims, **characterized in that** the identification and analysis in method step b) is carried out using methods selected from the group of molecular biological methods, protein analyses, assays for determining functionality and combinations thereof.

7. The method according to one of the preceding claims, **characterized in that** the influence of compounds on the proteins having stem cell activity identified in method step b) is examined in an additional method step c).

8. The method according to claim 7, **characterized in that** the influence of the at least one compound is examined in method step c) using methods selected from the group of molecular biological methods, protein analyses, assays for determining functionality and combinations thereof.

## Revendications

1. Procédé in vitro permettant l'identification et l'analyse de protéines à fonction de cellules souches dans la glande sudoripare humaine, comprenant les étapes de procédé suivantes :
a) mise à disposition d'au moins un équivalent de glande sudoripare tridimensionnel, comprenant de 500 à 500 000 cellules de glande sudoripare, l'au moins un équivalent de glande sudoripare tridimensionnel présentant un diamètre de 100 à 6 000 µm et l'au moins un équivalent de glande sudoripare tridimensionnel étant exempt de composés matriciels et de supports,
b) identification et analyse d'au moins une protéine à fonction de cellules souches dans l'au moins un équivalent de glande sudoripare tridimensionnel mis à disposition à l'étape a) du procédé.

2. Procédé selon la revendication 1, **caractérisé en ce que** l'au moins un équivalent de glande sudoripare tridimensionnel mis à disposition à l'étape a) du procédé présente un diamètre de 100 à 4 000 µm, de préférence de 100 à 2 000 µm, en particulier de 200 à 1 500 µm.

3. Procédé selon l'une des revendications précédentes, **caractérisé en ce que** l'au moins un équivalent de glande sudoripare tridimensionnel mis à disposition à l'étape a) du procédé est un équivalent de glande sudoripare tridimensionnel de la glande sudoripare humaine eccrine et/ou apocrine.

4. Procédé selon l'une des revendications précédentes, **caractérisé en ce que** l'au moins un équivalent de glande sudoripare tridimensionnel mis à disposition à l'étape a) du procédé contient au moins un type de cellule choisi dans le groupe des (i) cellules de queue, en particulier des cellules claires, des cellules sombres ainsi que des cellules myoépithéliales, des (ii) cellules de conduit ainsi que (iii) leurs mélanges.

5. Procédé selon l'une des revendications précédentes, **caractérisé en ce que** l'au moins une protéine à fonction de cellules souches à l'étape b) du procédé est choisie dans le groupe constitué par les protéines structurelles, les protéines de signalisation, les protéines de prolifération cellulaire, les protéines d'adhésion cellulaire ainsi que leurs mélanges.

6. Procédé selon l'une des revendications précédentes, **caractérisé en ce que** l'identification et l'analyse sont effectuées à l'étape b) du procédé à l'aide de méthodes choisies dans le groupe constitué par les méthodes de biologie moléculaire, les analyses de protéines, les dosages destinés à déterminer la fonctionnalité ainsi que leurs combinaisons.

7. Procédé selon l'une des revendications précédentes, **caractérisé en ce que** l'influence de composés sur la protéine à fonction de cellules souches identifiée à l'étape b) du procédé est étudiée dans une étape supplémentaire c) du procédé.

8. Procédé selon la revendication 7, **caractérisé en ce que** l'influence de l'au moins un composé à l'étape c) du procédé est effectuée à l'aide de méthodes choisies dans le groupe constitué par les méthodes de biologie moléculaire, les analyses de protéines, les dosages destinés à déterminer la fonctionnalité ainsi que leurs combinaisons.
